# EUROPEAN PATENT SPECIFICATION

(11) **EP 0 298 122 B1**
(45) Date of publication and mention of the grant of the patent: **15.09.1993**
(21) Application number: 87907339.3
(22) Date of filing: 04.11.1987
(51) Int. Cl.: A61B 5/00, A61B 10/00

(54) **LIVER FUNCTION INSPECTION APPARATUS**
GERÄT ZUR UNTERSUCHUNG DER LEBERFUNKTION
APPAREIL DE CONTROLE DES FONCTIONS HEPATIQUES

(30) Priority: 05.11.1986 JP 263046/86; 13.07.1987 JP 175517/87
(43) Date of publication of application: 11.01.1989
(73) Proprietor: SUMITOMO ELECTRIC INDUSTRIES, LTD., Osaka-shi, Osaka 541 (JP)
(72) Inventor: KANDA, Masahiko Osaka Works of Sumitomo, Konohana-ku Osaka-shi Osaka 554 (JP); AWAZU, Kunio Osaka Works of Sumitomo, Konohana-ku Osaka-shi Osaka 554 (JP)
(74) Representative: Patentanwälte Kirschner & Grosse
(86) International application number: PCT/JP87/00851
(87) International publication number: WO 88/03386

(56) References cited:
- WO-A-82/01948
- US-A- 3 677 648
- US-A- 4 017 192
- US-A- 4 602 641

## Description

### TECHNICAL FIELD

The present invention relates to a liver function testing apparatus. More specifically, it relates to a liver function testing apparatus for automatically performing measurement for testing/diagnosing liver function by injecting specific color dye, which is selectively taken in and removed by only the liver, into blood and measuring a blood plasma disappearance rate and a retention rate thereof.

### BACKGROUND ART

In general, the blood plasma disappearance rate and the retention rate have been measured by a method of blood collection through use of indocyanine green (hereinafter referred to as ICG) serving as specific dye. According to this method, intravenous injection of ICG is given to a testee to perform blood collection three times after lapses of five, ten and 15 minutes from the injection, and blood serum is separated upon coagulation of a blood clot so that absorbance at a wavelength of 805 nm is measured through a spectrophotometer to obtain ICG concentration values in the blood serum after the lapses of five, ten and 15 minutes from a previously obtained calibration curve (corresponding ICG concentration in blood vs. absorbance), thereby to calculate the blood plasma disappearance rate and the retention rate. In recent years, widely employed is a method of changing the quantity of ICG injection to measure the blood plasma disappearance rate several times thereby to obtain index expressing an amount of hepatic cell function R_{MAX} (removed maximal).

Japanese Patent Publication Gazette No. 58649/1985 has already proposed a method of measuring the blood plasma disappearance rate and the retention rate without performing blood collection. According to this method, light is applied through the body surface of an organism, which in turn transmits light of a wavelength having high ICG absorption sensitivity and light of a wavelength having substantially no ICG absorption sensitivity. The respective quantities of transmitted light are measured to obtain the blood plasma disappearance rate and the retention rate from time change (dye disappearance curve) of the light quantities.

In the aforementioned first method of blood collection, it is necessary to correctly measure the blood collection time after injection. However, the time has not been accurately measured in an actual test, and the operation for such measurement has been complicated. Further, the testee has been subjected to heavy mental and physical burdens by blood collection. In addition, the index R_{MAX} measuring method of measuring the blood plasma disappearance rate several times by changing the quantity of ICG injection requires blood collection over ten times, whereby the burdens on the testee are further increased.

According to the second measuring method without blood collection as disclosed in Japanese Patent Publication No. 58649/1985, the output of a sensor actually attached to an organism is fluctuated by influence such as blood flow disturbance caused by suppression on a blood vessel, vibration of the organism, being the object of measurement, pulsation in the organism, change of blood volume in the vital tissue (the blood volume in each part in the vital tissue is changed by merely vertically moving an arm) etc., whereby a correct dye disappearance curve cannot be obtained. Consequently, the blood plasma disappearance rate and the retention rate obtained by the curve cannot be recognized as being correct.

### DISCLOSURE OF THE INVENTION

Accordingly, a principal object of the present invention is to provide a liver function testing apparatus which can remove artifacts such as blood flow disturbance, vibration of an organism, pulsation in the organism and change of the blood volume in the vital tissue, to enable correct measurement.

For achieving the above object, the present invention provides a liver function testing apparatus for testing liver function as defined in claim 1.

In a preferred embodiment of such a liver function testing apparatus, a coefficient of a simulation function serving as a function of time is obtained by using the method of least squares on the basis of the operated value correlated with the specific dye concentration.

In a more preferred embodiment of the present invention, a blood plasma disappearance rate of the specific dye is found on the basis of the obtained coefficient of the simulation function.

In a further preferred embodiment of the present invention, a retention rate of the specific dye in a prescribed period is found on the basis of the obtained coefficient of the simulation function.

In a further embodiment of the present invention, index expressing an amount of hepatic cell function R_{MAX} is found on the basis of the obtained coefficient of the simulation function.

Further preferred embodiments of the present invention are defined in the subclaims.

Thus, according to the present invention, first light of a wavelength absorbed by specific dye dosed into blood of vital tissue to be taken in and removed from the liver and second light of a wavelength not absorbed by the dye are applied to the vital tissue and first and second photoelectric conversion signals corresponding to the first light and the second light obtained from the vital tissue are sampled so that a coefficient of a regression line expression between the first and second photoelectric conversion signals is determined on the basis of variable components in the blood included in the sampled first and second photoelectric conversion signals to perform biocalibration, in order to operate a value correlated with specific dye concentration in the blood on the basis of a sampling signal during a prescribed period after a lapse of a predetermined time from injection of the specific dye and the decided coefficient of the regression line expression. Thus, artifacts such as blood flow disturbance, vibration and pulsation of an organism etc. in attachment of a sensor to an organism can be removed by biocalibration, to operate the value correlated with the specific dye concentration. Thus, correct time management of a disappearance curve of the specific dye is enabled, to obtain correct data. Further, the blood plasma disappearance rate, the retention rate, an index expressing the total amount of hepatic cell function etc. can be more correctly expressed not from several samples by the conventional blood correction method but from a larger number of data of the disappearance curve, thereby to improve reliability of the data.

These and other objects, features, aspects and advantages of the present invention will become more apparent from the following detailed description of the present invention will become more apparent from the following detailed description of the present invention when taken in conjunction with the accompanying drawings.

### [Brief Description of the Drawings]

Figs. 1 to 4 are diagrams for illustrating the principle of the present invention;
Fig. 5 is a schematic block diagram showing the structure of an embodiment of the present invention;
Fig. 6 illustrates timing for detecting quantities of light of wavelengths λ₁ and λ₂ after passage through a prescribed optical path in a measured object;
Fig. 7 illustrates data stored in a RAM as shown in Fig. 1;
Figs. 8A to 8D are flow charts for concretely illustrating the operation of the embodiment, in which Fig. 8A shows a data sampling subroutine, Fig. 8B shows a biocalibration mode, Fig. 8C shows an initialization mode and Fig. 8D shows a measurement mode;
Figs. 9 to 12 are illustrative of exemplary displays on a display part as shown in Fig. 5;
Fig. 13 shows an example of a disappearance curve of specific dye measured by the present invention;
Figs. 14 and 15 illustrate result of light quantity data L₁ and L₂, a disappearance curve, a blood plasma disappearance rate and 15-minute retention rate measured through the present invention;
Figs. 16 to 19 are diagrams for illustrating effects by the present invention;
Fig. 20 illustrates data stored in a RAM employed in another embodiment of the present invention;
Figs. 21A and 21B are flow charts for illustrating operation of a measurement mode in another embodiment of the present invention; and
Figs. 22 to 24 are diagrams for illustrating operation of another embodiment of the present invention.

### THE BEST MODE FOR CARRYING OUT THE INVENTION

Before explaining embodiments of the present invention, description is now made on the principle of biocalibration employed in the present invention.

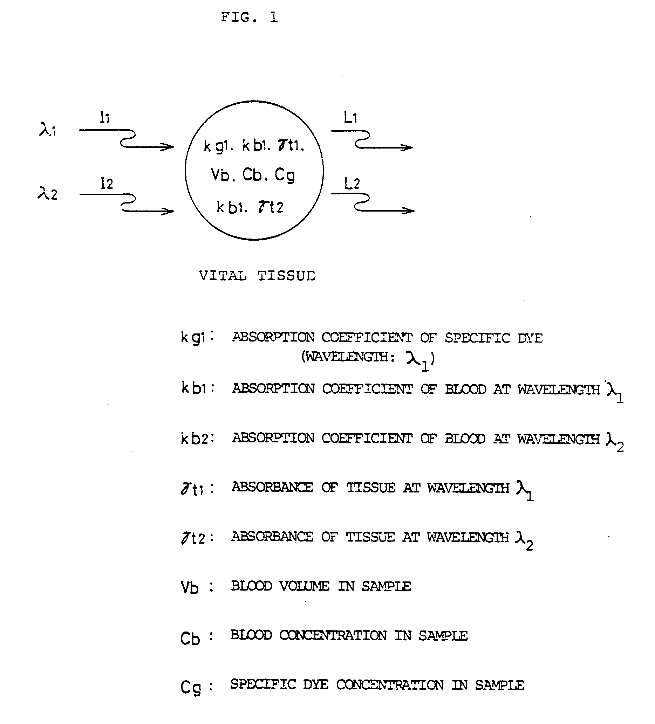

Figs. 1 to 4 are diagrams for illustrating the principle of the biocalibration in the present invention.

It is assumed that symbols I₁ and I₂ indicate quantities of light having a wavelength λ₁ which is largely absorbed by specific dye and light of a wavelength λ₂ which is not absorbed by the specific dye incident upon vital tissue, and symbols L₁ and L₂ indicate light quantities after passage through a prescribed optical path in the vital tissue. Relations between the incident light quantities I₁ and I₂ and the passing light quantities L₁ and L₂ in injection of the specific dye are as follows:

$\text{(1) logI₁/L₁ = kg₁ · Cg · Vb + f₁(Cb, Vb) + γt₁}$

$\text{(2) logI₂/L₂ = f₂(Cb, Vb) + γt₂}$

Respective coefficients and variables are shown in Fig. 1. Symbols f₁ and f₂ represent functions which are determined by characteristics of blood at the wavelengths λ₁ and λ₂.

On the other hand, relations between the incident light quantities I₁ and I₂ and the passing light quantities L₁ and L₂ before injection of the specific dye are as follows:

$\text{(3) logI₁/L₁ = f₁(Cb, Vb) + γt₁}$

$\text{(4) logI₂/L₂ = f₂(Cb, Vb) + γt₂}$

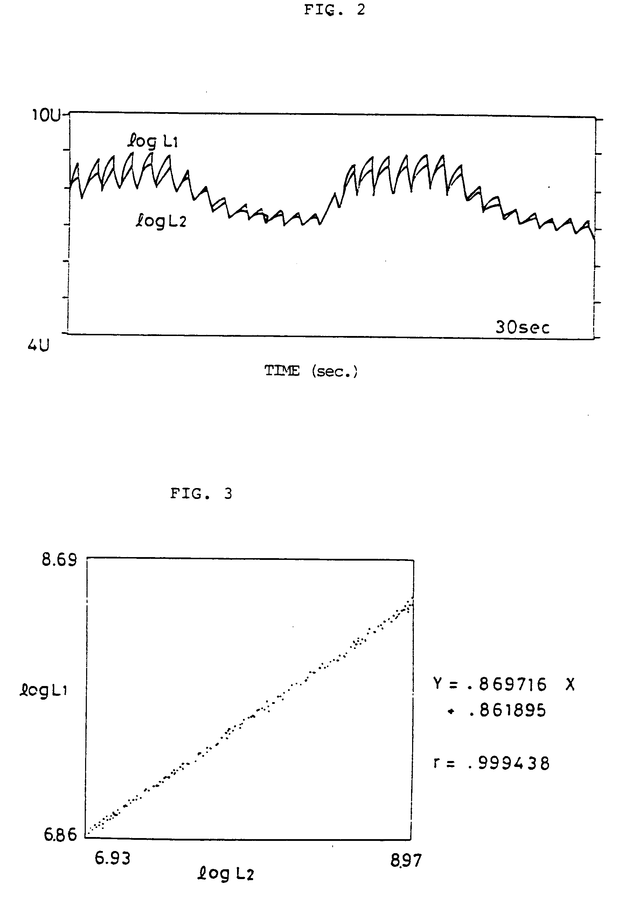

The relation between the passing light quantities L₁ and L₂ previous to actual injection of the specific dye is measured as shown Fig. 2, to be in the linear relation as shown in Fig. 3. This is the data in case of attaching a sensor to an organism and fluctuating the blood volume in the organism. It has been confirmed that such linearity has reproducibility, with no individual difference.

Then, the expressions (3) and (4) would appear as follows:

$\text{(5) logL₁ = AlogL₂ + B}$

That is, the same can be expressed as follows, by using the expressions (3) and (4):

$\text{(6) logI₁ - {f₁(Cb, Vb) + γt₁} = A[logI₂ - {f₂(Cb, Vb) + γt₂}] + B}$

where Cb represents blood concentration in a sample and Vb represents blood volume in the sample.

A function C obtained by multiplying concentration of the specific dye by the blood volume in the sample and the absorption coefficient of the specific dye by using the expressions (1) and (2) after injection of the specific dye can be expressed as follows:

$\text{(7) C = logL₁ - [AlogL₂ + B]}$

The function C is found by the expression (7) as follows:

$\text{(8) C = logI₁ - kg · Cg · Vb - f₁(Cb, Vb) + γt₁ - A[logI₂ - {f₂(Cb, Vb) + γt₂}] - B}$

Through the expression (6), we have:

$\text{(9) C = - kg · Cg · Vb}$

Hence, it is understood that a signal of the function C can be obtained by using Fig. 3 as a biocalibration curve.

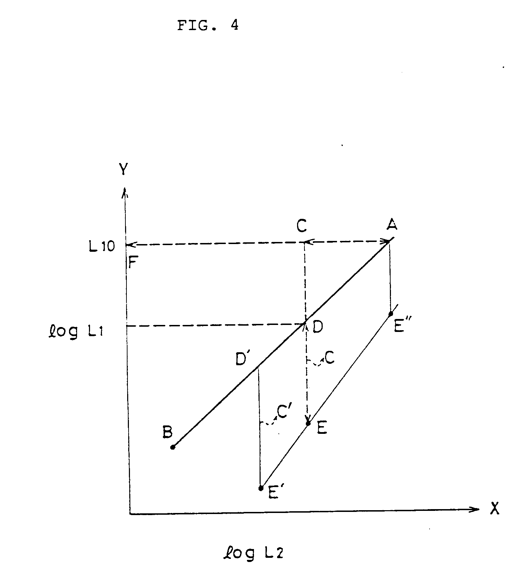

As to the function C, however, although the coefficient kg is constant, it can be considered that the blood volume Vb in each part is changed from time to time, and hence, if the blood volume Vb in a prescribed sample created by the sensor once attached is changed, the amount of the specific dye is also changed in proportion thereto although the dye concentration remains unchanged. This is typically shown in Fig. 4.

Referring to Fig. 4, it is assumed that D̅E̅ represents the value of the function C after a lapse of t₁ minutes. The blood contained in the prescribed sample obtained after a lapse of t₁ + Δt minutes is changed in volume, whereby an observation point is changed from E to E'. Assuming that Δt is sufficiently less than one minute, the specific dye concentration in the blood after the lapse of t₁ minutes may be considered identical to that after the lapse of t₁ + Δt minutes. However, as to the function C, the change is from C = D̅E̅ to C' = D̅'̅E̅'̅. C ≠ C', and hence some correction must be performed. Hence, by normalizing D̅E̅ and D̅'̅E̅'̅ at the point L₁₀, apparent fluctuation of the dye concentration due to the fluctuation of the blood volume can be corrected. When the specific dye is injected, a signal of only logL₁ is fluctuated, to lie to a point E, for example. At this time, D̅E̅ becomes the function C as shown in the expression (9). The blood volume Vb in the expression (9) can be interpreted as being denoted by C̅D̅, and hence, normalizing the Y coordinate of a point A as L₁₀, the same is expressed as follows: 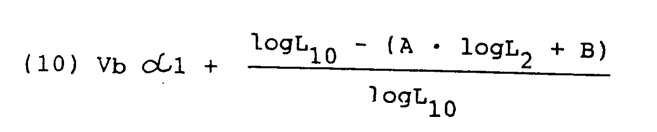
Hence, a signal Cg corresponding to the specific dye concentration can be found by the expressions (7) and (10) as follows: 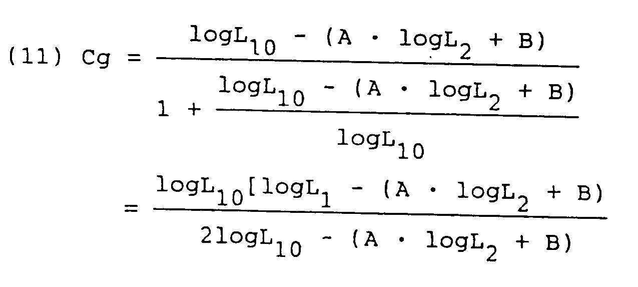
Using the method of least squares, the function Cg of a simulation curve in time change of the aforementioned result Cg of calculation is expressed as follows: 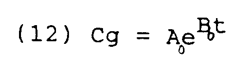
where t represents the elapsed time after injection of the specific dye and symbols A and B represent constants.

The constants A and B are found by the above expression (12). The blood plasma disappearance rate k and the T-minute retention rate R % are expressed as follows:

${\text{(13) k = -B}}_{\text{o}}$ 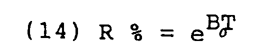
where T represents the elapsed time after injection characteristically expressing intake of the specific dye into the liver.

While the biocalibration employed in the present invention has been described in the above, description is now made on an embodiment of the present invention employing the aforementioned biocalibration.

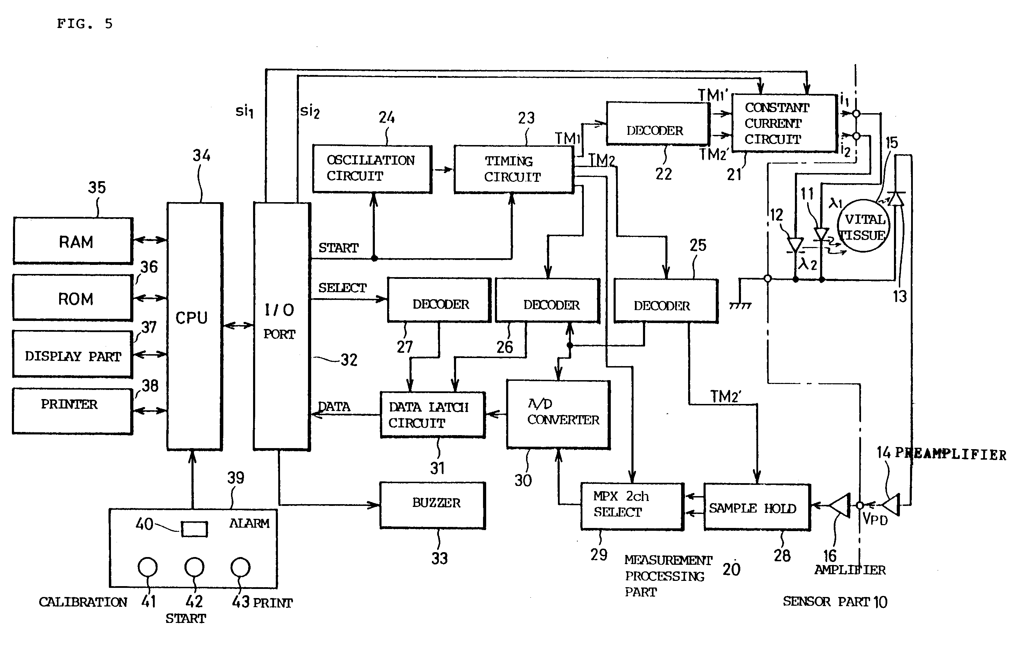

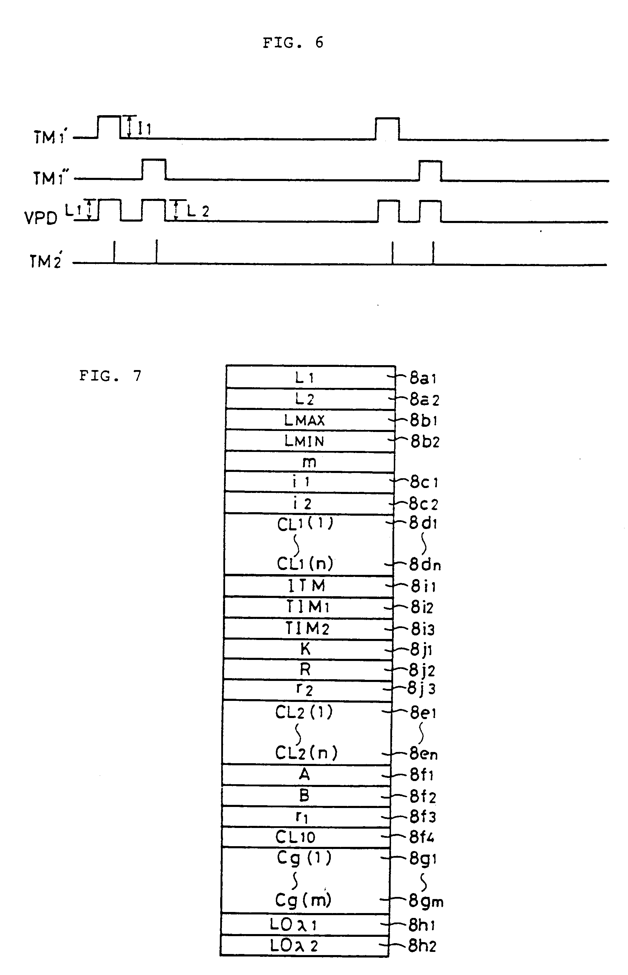

Fig. 5 is a schematic block diagram showing an embodiment of the present invention, Fig. 6 is a timing chart for detecting quantities of light of wavelengths λ₁ and λ₂ after passage through a prescribed optical in a measured object, and Fig. 7 illustrates data stored in a RAM as shown in Fig. 5.

Referring to Fig. 5, a liver function testing apparatus is formed by a sensor part 10 and a measurement processing part 20. The sensor part 10 includes a first light source 11, a second light source 12, a light receiving element 13 and a preamplifier 14. The first light source 11 and the second light source 12 generate optical pulses of a wavelength λ₁ having large absorbance to specific dye and optical pulses of a wavelength λ₂ having no absorbance, respectively. The light receiving element 13 receives light applied to vital tissue 15 from the light sources 11 and 12 to pass through a prescribed optical path. The light sources 11 and 12 are driven by the measurement processing part 20 to alternately emit light by pulse operation, respectively.

The measurement processing part 20 includes a CPU 34 which operates as arithmetic means. The CPU 34 supplies a start signal to an oscillation circuit 24 and a timing circuit 23 through an I/O port 32. The oscillation circuit 24 regularly oscillates to produce a prescribed clock signal. This clock signal and the aforementioned start signal are utilized to supply constant currents i₁ and i₂ to the first light source 11 and the second light source 12 from a constant current circuit 21 through the timing circuit 23 and a decoder 22 at timing TM₁' and TM₁'' in Fig. 6.

The light emitted from the first light source 11 and the light emitted from the second light source 12 pass through the prescribed optical path in the vital tissue 15, to be incident upon the light receiving element 13. A current generated from the light receiving element 13 is supplied to the preamplifier 14 to be subjected to current-to-voltage conversion, while being amplified to be supplied to the measurement processing part 20. Output of the preamplifier 14 is amplified to a level within a prescribed range by an amplifier 16 provided in the measurement processing part 20, whereby output such as V_{PD} in Fig. 6 is obtained. A sample and hold circuit 28 samples and holds output from the amplifier 16 on the basis of a timing signal TM2', shown in Fig. 6, generated by the timing circuit 23 and a decoder 25.

The signal thus sampled and held is selected by a multiplexer 29 and converted into a digital signal by an A-D converter 30, to be data-latched by a data latch 31. At this time, the multiplexer 29, the A-D converter 30 and the data latch 31 are controlled in timing by the timing circuit 23 and the decoder 26.

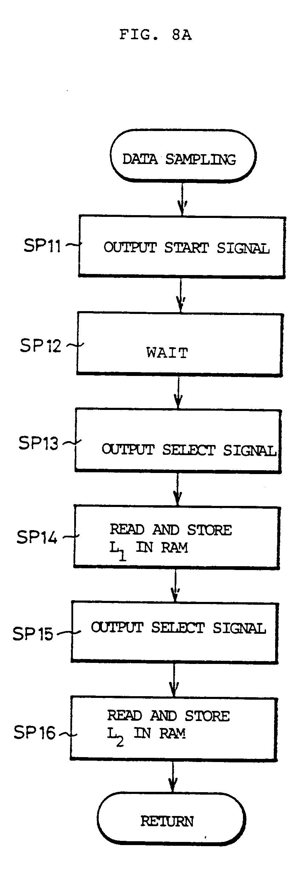

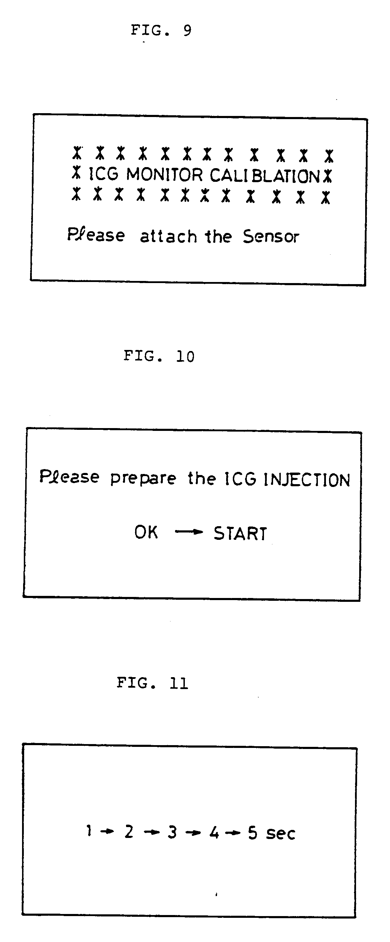

The latched data are timed by a decoder 27 through a select signal outputted from the CPU 34 through the I/O port 32, to be taken in a RAM 35 as digital signals L₁ and L₂. The I/O port 32 is connected with a buzzer 33, which informs timing for injecting the specific dye. Further, the CPU 34 is connected with the RAM 35, a ROM 36, a display part 37 and a operation part 28. The RAM 35 is adapted to store data as shown in Fig. 7 as hereinafter described, and the ROM 36 stores programs based on flow charts as shown in Figs. 8A to 8D as hereinafter described. The display part 37 displays data as shown in Figs. 9 to 12, as hereinafter described. A printer 38 is adapted to print the result of a liver function test.

The function part 39 includes an alarm LED 40, a calibration key 41, a start key 42 and a print key 43. The alarm LED 40 is adapted to display an alarm when reliability of the test result is small and the calibration key 41 is adapted to set a biocalibration mode, while the start key 42 is adapted to command start of a measurement mode and the print key 43 is adapted to command printout of the test result.

In the aforementioned exemplary structure as shown in Fig. 5, the light emitted from the first and second light sources 11 and 12 to pass through the prescribed optical path in the vital tissue 15 is received by a single light receiving element 13. However, such means is not restricted to this but light receiving elements may be provided in correspondence to the first and second light sources 11 and 12 respectively to sample outputs of the respective light receiving elements, thereby to read the respective sampling outputs by the CPU 34 in a time-sharing manner. Alternatively, a single light source commonly emitting light having a wavelength λ₁ absorbed by specific dye and light having a wavelength λ₂ not absorbed by the same may be provided as light source means, with provision of two filters for individually transmitting the light of the respective wavelengths and light receiving elements corresponding to the respective ones of the filters.

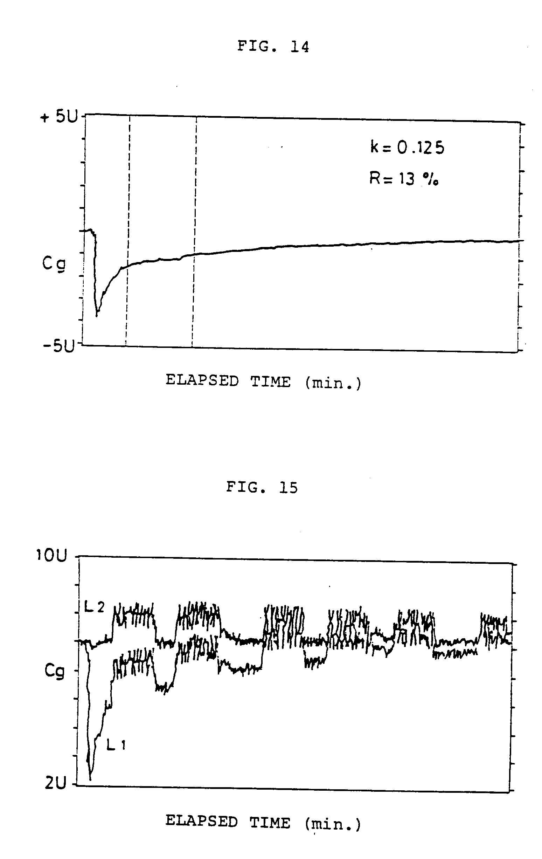

Fig. 7 illustrates data stored in the RAM 35 as shown in Fig. 5 and Figs. 8A to 8D are flow charts for illustrating concrete operation of the embodiment of the present invention, while Figs. 9 to 12 are illustrative of exemplary displays on the display part as shown in Fig. 5, Fig. 14 is illustrative of an exemplary disappearance curve of specific dye, and the blood plasma disappearance rate k and the T-minute retention rate R % measured by the present invention.

With reference to Figs. 5, 8A to 8D and 14, description is now made on the concrete operation of the embodiment of the present invention.

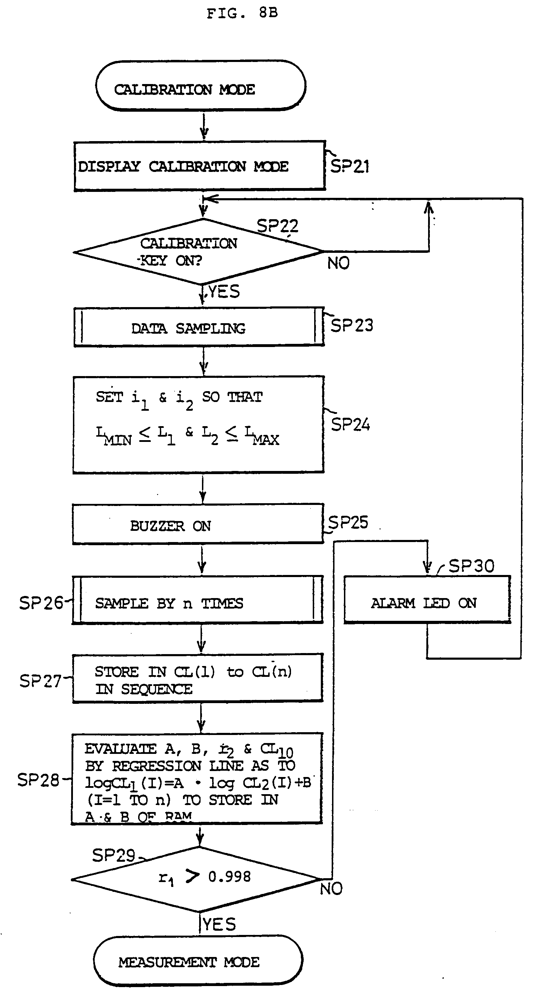

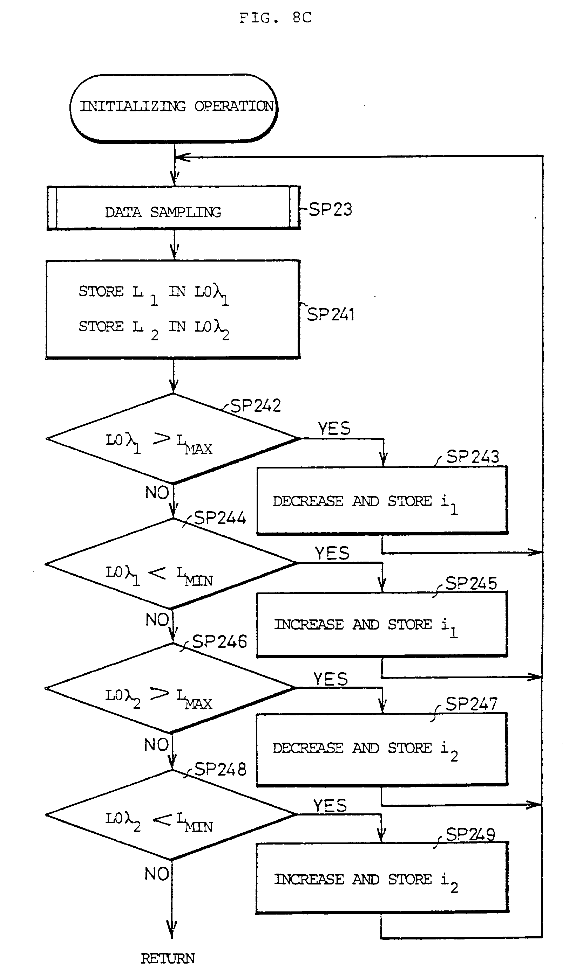

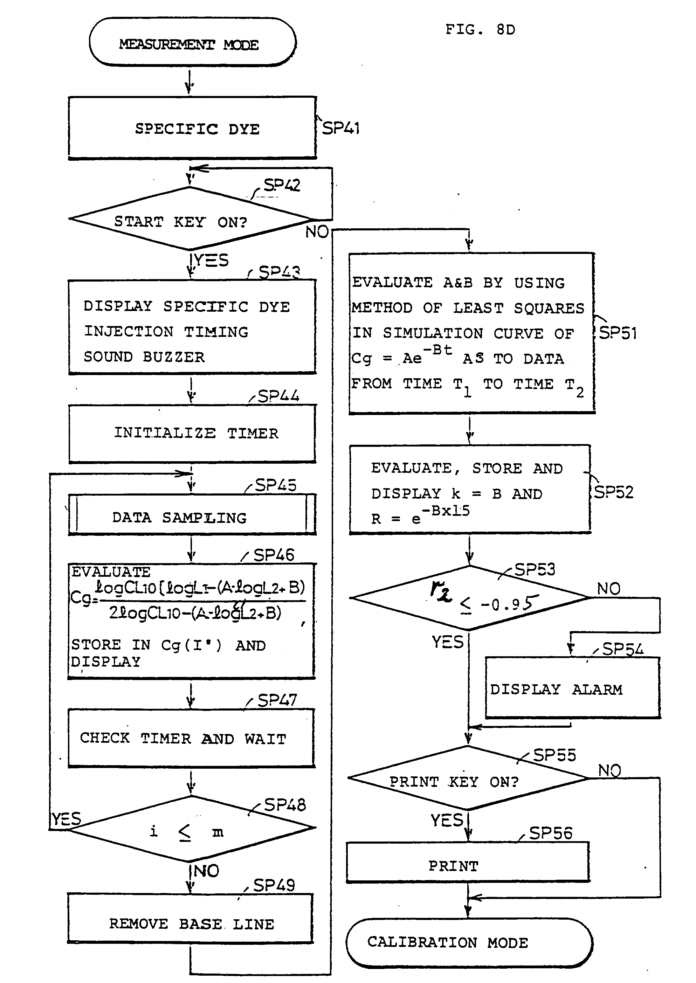

The operation of the inventive apparatus includes a data sampling mode, a biocalibration mode, an initialization mode and a measurement mode, and Figs. 8A, 8B, 8C and 8D show operation flows in these modes respectively.

First, it is pointed out that the data sampling mode as shown in Fig. 8A is executed as subroutines in the calibration mode and the measurement mode as hereinafter described. Steps (abbreviated as SP in the figures) SP11 to SP16 are adapted to sample quantities of light of a pair of wavelengths λ₁ and λ₂ after passage through a measured object and store the same in the RAM 35. Namely, the CPU 34 outputs the start signal from a line as shown in Fig. 5 through the I/O port 23 at the step SP11. The values L₁ and L₂ are data-latched by the start signal, as hereinabove described. The CPU 34 waits until the data are latched at the step SP12.

Then, at the step SP13, the CPU 34 outputs the select signal to a select line as shown in Fig. 5 through the I/O port 32, to read the data of L₁ through the I/O port 32 at the step SP14, thereby to store the same in a storage area 8a1 of the RAM 35 as shown in Fig. 7.

Similarly, the CPU 34 stores the data of L₂ in a storage area 8a2 of the RAM 35 at the steps SP15 and SP16.

Upon completion of the aforementioned operation at the step SP16, the CPU 34 returns to the original step. This will be described with reference to Fig. 8B showing the biocalibration mode and Fig. 8D showing the measurement mode.

Fig. 8B shows the operation flow chart of the biocalibration mode, which is started upon power supply to the apparatus or upon completion of the operation of the measurement mode as shown in Fig. 8D, as hereinafter described. At a step SP21, the CPU 34 makes the biocalibration mode appear on the display part 37. This display shows that the apparatus enters the biocalibration mode and indicates mounting of the sensor part 10 as shown in Fig. 9, for example. In accordance with this indication, an operator attaches the sensor part 10 to the vital tissue 15.

Thereafter the CPU 34 waits until the calibration key 41 is operated at a step SP22. When the calibration key 41 is operated, the CPU 34 advances to a step SP23, to execute the data sampling subroutine as shown in Fig. 8A, as hereinabove described.

Then, the CPU 34 controls the constant current circuit 21 as shown in Fig. 5 so that the data L₁ and L₂ read at the step SP23 are within ranges of light quantity data L_{MAX} and L_{MIN} stored in storage areas 8b1 and 8b2 of the RAM 35. The CPU 34 then stores current set values i₁, i₂ in storage areas 8c1 and 8c2 in the RAM 35. Thereafter the currents i₁, i₂ regularly flow to the light sources 11 and 12. Initializing operation for the aforementioned currents will be described in further detail with reference to Fig. 8C.

Then, the CPU 34 sounds the buzzer at a step SP25, to inform that power setting is completed. Subsequent steps SP26 to SP29 are shown as a flow chart for performing the aforementioned biocalibration. In more concrete terms, the CPU 34 samples the values of L₁ and L₂ n times respectively at the steps SP26 and SP27, to make CL₁(1) to CL₁(n) stored in storage areas 8d1 to 8dn and CL₂(1) to CL₂(n) stored in storage areas 8e1 to 8en. At the subsequent step SP28, the CPU 34 performs regression line analysis with respect to log CL₁(I) and logCL₂(I) (I = 1 to n), in accordance with the following operation expression:

$\text{logCL₁(I) = A logCL₂(I) + B}$

The CPU 34 finds the values A and B in the above operation expression, a correlation coefficient r₁ and the maximum value of CL₁(I) (I = 1 to n) as CL₁₀, to store the same in storage areas 8f1, 8f2, 8f3 and 8f4 in the RAM 35 respectively.

Then, at the step SP29, the CPU 34 determines whether or not the correlation coefficient r₁ is at least 0.998 in order to verify reliability of the biocalibration, advances to a step SP30 if the same is less than 0.998 to light the alarm LED 40, and returns to the step SP22 to again perform biocalibration. On the other hand, if a determination is made that the correlation coefficient r₁ is at least 0.998, the CPU 34 advances to the measurement mode as shown in Fig. 8D. The reference value 0.998 of the correlation coefficient r₁ herein employed is a mere example, which is determined by performance of the entire apparatus. During the data sampling by n times at the step SP26, the testee raises and brings down his hand and suppresses the same by the sensor, in order to change the blood volume in the organism.

With reference to Fig. 8C, the aforementioned initializing operation at the step SP24 as shown in Fig. 8B will now be described in more concrete terms.

The light quantity data L₁ and L₂ of the light of the wavelengths λ₁ and λ₂ are stored in the storage areas 8a1 and 8a2 of the RAM 35. At a step SP241, the CPU 34 makes the values of L₁ and L₂ stored in storage area 8h1 and 8h2 in the RAM 35 as L0λ₁ and L0λ₂ respectively. Then the CPU 34 executes steps SP242 to SP249, to adjust the set values of the currents flowing from the constant current circuit 21 so that L0λ₁ and L0λ₂ are set between the light quantity data L_{MAX} and L_{MIN} (L_{MAX} > L_{MIN}) stored in the storage areas 8b1 and 8b2 of the RAM 35.

In more concrete terms, if L0λ₁ is greater than L_{MAX} at the step SP242, the CPU 34 advances to the step SP243 to set the current set value i₁ at a small value to again execute the steps SP23 and SP241, and a determination is again made as to whether or not L0λ₁ is greater than L_{MAX} at the step SP242. If L0λ₁ is less than L_{MAX}, the CPU 34 advances to the step SP244 to determine whether or not L0λ₁ is less than L_{MIN}. If L0λ₁ is less than L_{MIN}, the CPU 34 increases the value of the current set value i₁ at the step SP245, to return to the aforementioned step SP23. This operation is repeated to set the current set value i₁ so that L0λ₁ is between L_{MAX} and L_{MIN}.

Then, at the steps SP246 to SP249, the current set value i₂ is set so that L0λ₂ is between L_{MAX} and L_{MIN}, similarly to the steps SP242 to SP245. Thus, the current set values i₁ and i₂ finally set at the steps SP23 to SP249 are stored in the storage areas 8c1 and 8c2 of the RAM 35.

With reference to Fig. 8D, description is now made on the measurement mode. At a step SP41, the CPU 34 makes a display for injection of the specific dye on the display part 37. As to this display, indication is made for injection of the specific dye such as ICG as shown in Fig. 10, for example. In accordance with the display, the operator prepares for injection of the specific dye to the testee. At a step SP42, the CPU 34 waits until the start key 42 is operated. Upon a determination that the start key 42 is operated, the CPU 34 displays timing for injecting the specific dye at a step SP43, while sounding the buzzer 33. This is displayed as 1 → 2 → 3 → 4 → 5 as shown in Fig. 11, for example, so that the measurer injects the specific dye upon display of "5". The CPU 34 generates a first sound from the buzzer 33 with the displays of "1", "2", "3" and "4", while generating a different sound from the buzzer 33 upon display of "5".

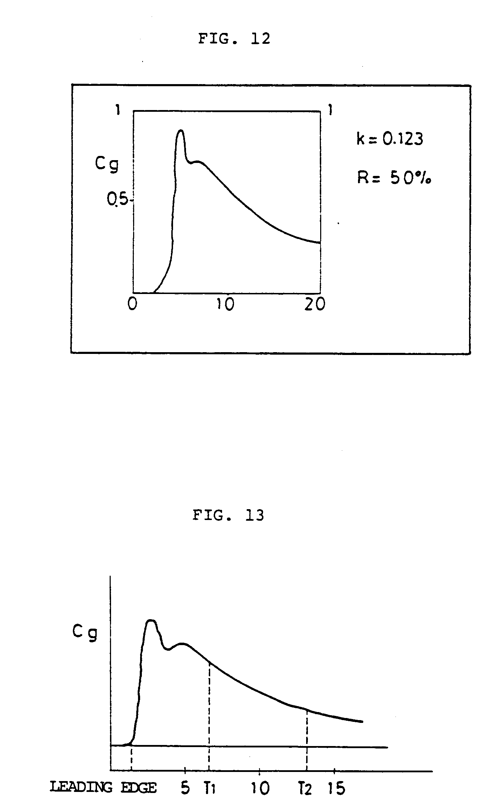

Upon generation of the sound and the display, the measurer injects the specific dye. The CPU 34 sets "0" as the initial value of a timer at a step SP44. Then, at a step SP45, the CPU 34 executes a data sampling program, which is the subroutine as hereinabove described with reference to Fig. 8A. Then, the sampling data are stored in the storage areas 8a1 to 8a2 of the RAM 35 as L₁ to L₂, respectively. At a step SP46, the CPU 34 performs operation based on the following operation expression by using the coefficients A, B, and CL₁₀ stored in the storage areas 8f1, 8f2 and 8f4 of the RAM 35 in the biocalibration mode as hereinabove described with reference to Fig. 8B, to store Cg(I) in a storage area 8g1 of the RAM 35: 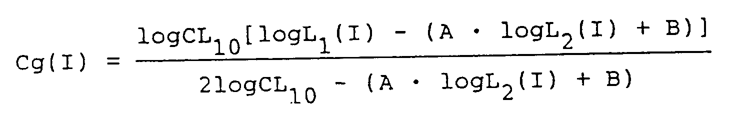
The value of Cg(I) is displayed on the display part 37 at the step SP46 in a mode as shown in Fig. 12, for example. Referring to Fig. 12, the axis of abscissa indicates the elapsed time from injection of the specific dye and axis of ordinate indicates the value of Cg(I). Assuming that m represents the sampling number of a disappearance curve of the specific dye, symbol I indicates integers 1 to m, and assuming that Tₛ represents a measuring time of the disappearance curve, a single sampling time is ${\text{ITM = T}}_{\text{s}} \text{/(m - 1)}$ . The same coincides with the injection time of the specific dye in the case of I = 1, as a matter of course. At a step SP47, the CPU 34 waits during this sampling time ITM.

Upon a lapse of this standby time, the CPU 34 judges whether or not i is greater than m at a step SP48. The CPU 34 advances to a step SP49 if i is greater than m, while the same again returns to the step SP45 to repeat sampling if the former is less than the latter. The data Cg(I) stored in the storage areas 8g1 to 8gm of the RAM 35 draw a disappearance curve of the specific dye as shown in Fig. 13, for example, and the leading edge thereof is detected so that data preceding thereto are subtracted as baselines from the respective values of Cg(I), to be again stored in the storage areas 8g1 to 8gm. Needless to say, L₁ to L₂ at the step SP45 may be average values of m times, in order to improve the accuracy of measurement.

Then, at a step SP51, the CPU 34 finds the constants A and B by using the method of least squares in a simulation curve of: 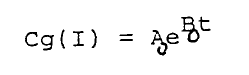
${\text{I = T}}_{\text{s}} \text{/(m - 1) (min.)}$

with respect to data between times T₁ to T₂ (0 < T₁ < T₂ <HS1504> Tₛ) within the data Cg(I) stored in the storage areas 8g1 to 8gm.

Then, the CPU 34 performs operation of the blood plasma disappearance rate ${\text{k = -B}}_{\text{o}}$ and the T-minute retention rate 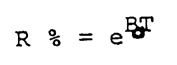
at a step SP52, to evaluate k and R %. The values k and R % thus evaluated are stored in storage areas 8j1 and 8j2 of the RAM 35, respectively. At this time, the CPU 34 operates a correlation coefficient r₂ by the method of least squares, to make the correlation coefficient r₂ thus operated stored in a storage area 8j3 of the RAM 35. The CPU 34 further generates an end sound from the buzzer 33.

Further, the CPU 34 makes the values k and R % appear on the display part 26 in a mode as shown in Fig. 12, for example. Then, at a step SP53, the CPU 34 determines whether or not the correlation coefficient r₂ is less than -0.95, for example. This determination is made to check the degree of correlation since correlation is improved as the correlation coefficient r₂ approaches -1. The value -0.95 is provisionally selected between zero and -1, and reliability of the apparatus is improved as the value comes closer to -1.

If the correlation coefficient r₂ is greater than 0.95, for example, the CPU 34 determines that reliability is insufficient to light the alarm LED 40 at the step SP54. On the other hand, if the correlation coefficient r₂ is less than -0.95, for example, at the step SP53, the CPU 34 advances to a step SP55 without flashing the alarm LED 44, since the measurement is reliable. At the step SP55, the CPU 34 determines whether or not the print key 43 is operated, to make the printer 38 print the values k and R % if the determination is of YES.

If necessary, the CPU 34 also makes characteristic dye disappearance curves of Cg(I) stored in the storage areas 8g1 to 8gn of the RAM 35 printed, to advance to the biocalibration mode as shown in Fig. 8B. Also when a determination is made that the print key 43 is not operated at the step SP55, the CPU 34 advances to the calibration mode.

Fig. 14 shows the result of a measurement experiment in the liver function testing apparatus as shown in Fig. 5. The sensor part 10 was attached on a left fingertip of a male patient having hepatic disease (age: 60, weight: 48 Kg), to intravenously inject an aqueous solution containing 24 mg of ICG (0.5 mg per Kg) from the vein of his right front elbow. Fig. 15 shows the time change of L₁, L₂ in case of employing a light emitting diode of wavelength λ₁ = 810 nm as the first light source 11 and a light emitting diode of wavelength λ₂ = 940 nm as the second light source 12.

The value k calculated by the ICG disappearance curve was 0.125 as shown in Fig. 14 and the value R % was 13 %, while the value k measured by the conventional blood collection method was 0.124 and the value R % was 12.8 %, substantially in coincidence. Fig. 15 also shows raw data of L₁ and L₂. It is clearly understood from Fig. 14 that the blood volume in the organism was fluctuated.

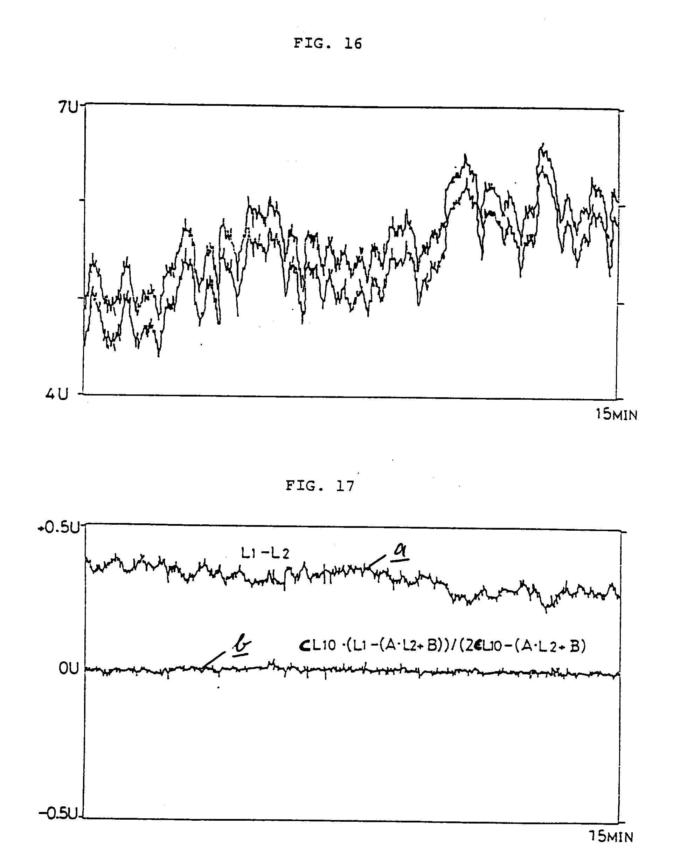

Figs. 16 to 19 show results of experiments for illustrating effects attained by the present invention.

Fig. 16 shows time changes for 15 minutes in intensity levels of the first light and the second light passing through the vital tissue 15 from the light sources 11 and 12 while bringing the vital tissue 15 in a rest state. Merely finding difference (L₁ - L₂) between the first light and the second light, the base line is largely fluctuated as characteristic a in Fig. 17. When the fluctuation of the blood volume is corrected by the biocalibration according to the present invention, the base line is substantially stable as shown by characteristic b in Fig. 17.

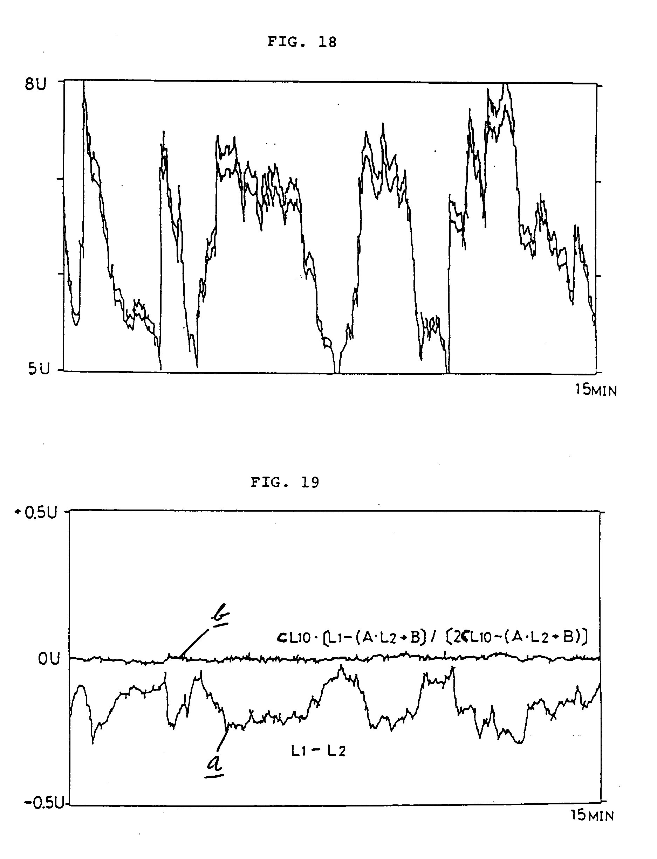

Fig. 18 shows time changes for 15 minutes in intensity levels of the first light and the second light passing through the vital tissue 15 from the light sources 11 and 12 when the testee's body is moved to largely fluctuate the blood volume. Evaluating difference between the first light and the second light with such large fluctuation, the base line is largely fluctuated as shown by characteristic a in Fig. 19. When the fluctuation of the blood volume is corrected by the biocalibration according to the present invention, the base line is substantially stabilized as shown by characteristic b in Fig. 19.

In the aforementioned embodiment, the present invention is applied to the case of evaluating the coefficient of the simulation function by using the method of least squares on the basis of the value correlated with the specific dye concentration in the blood thereby to evaluate the blood plasma disappearance rate k and the retention rate R %. However, the present invention is not restricted to this but further applicable to the case of obtaining index R_{MAX} on the basis of the aforementioned coefficient as obtained. Description is now made on such an embodiment.

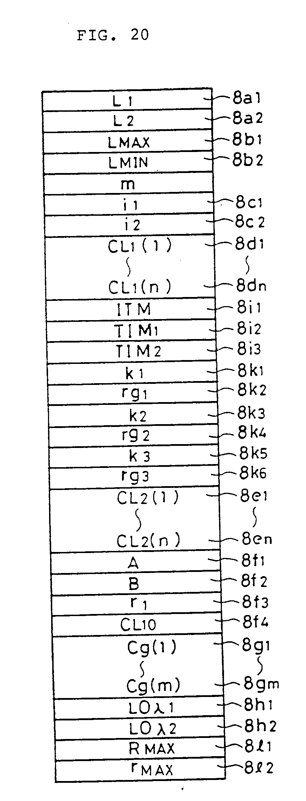

Fig. 20 illustrates data stored in a RAM provided in an apparatus for measuring index R_{MAX} and index r_{MAX}.

The apparatus for measuring index R_{MAX} and index r_{MAX} is identical in structure to that shown in Fig. 5, but the RAM 35 is provided with storage areas 8k1 to 8k6 and 8ℓ1 and 8ℓ2 as shown in Fig. 20, in place of the storage areas 8j1 to 8j3 as shown in Fig. 7.

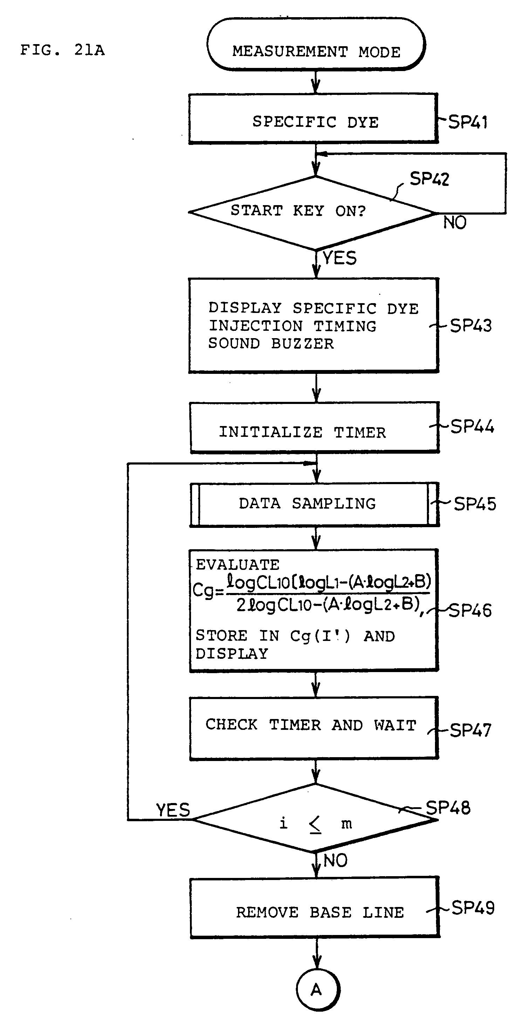

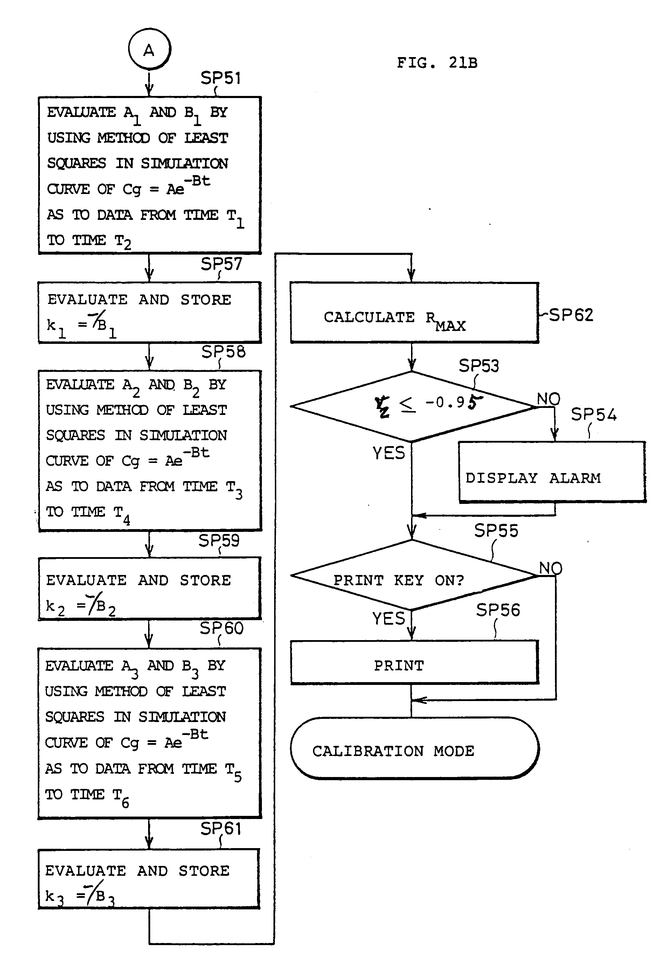

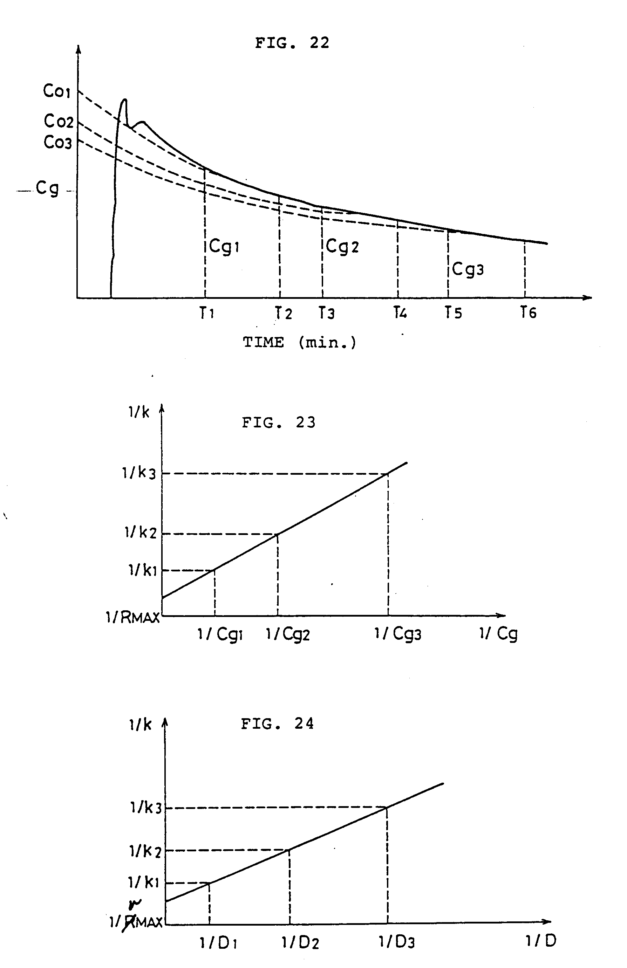

Figs. 21A and 21B are flow charts for illustrating a measurement mode for measuring index R_{MAX}, and Figs. 22 to 24 are diagrams for illustrating operation for measuring index R_{MAX} and index r_{MAX}.

A data sampling mode in measurement of index R_{MAX} is identical to that shown in Fig. 8A and a biocalibration mode is identical to that shown in Fig. 8B, while initializing operation is identical to that shown in Fig. 8C. Within the operation of the measurement mode as shown in Figs. 21A and 21B, steps SP41 to SP51 and SP53 to SP56 are identical to those shown in Fig. 8D, and hence redundant description is omitted.

In order to measure index R_{MAX} and index r_{MAX}, it is necessary to operate functions of simulation curves in time change of results of operation in at least two or more blocks by using the method of least squares, to evaluate coefficients k of specific dye as to respective blocks on the basis of the functions, as shown in Fig. 22.

Then, at a step SP51, a CPU 34 operates constants A₁ and B₁ in a block between times T₁ to T₂, similarly to the above embodiment. At a step SP57, the CPU 34 evaluates k₁ from k₁ = B₁ while evaluating a correlation coefficient r_{g1}, to store the same in the storage areas 8k1 and 8k2 of the RAM 35. Similarly, the CPU 34 evaluates constants A₂ and B₂ in a block between times T₃ and T₄ at a step SP58, and evaluates a coefficient k₂ and a correlation coefficient r_{g2} at a step SP59 to store the same in the storage areas 8k3 and 8k4. The CPU 34 further operates constants A₃ and B₃ at a step SP60 and evaluates a coefficient K₃ and a correlation coefficient r_{g3} at a step SP61, to store the same in the storage areas 8k5 and 8k6. Then the CPU 34 operates index R_{MAX} at a step SP62.

The times T₁ to T₆ and the coefficients k₁ to k₃ are mapped in relation as shown in Fig. 22. The CPU 34 assumes that Cg₁, Cg₂ and Cg₃ represent values corresponding to specific dye concentration values at the times T₁, T₃ and T₅, to display the graph as shown in Fig. 23. Referring to Fig. 23, the axis of abscissa is indicated by 1/Cg and the axis of ordinate is indicated by 1/K. On the basis of these data, the CPU 34 operates a and b by using the method of least squares, through the following operation expression:

${\text{1/k}}_{\text{i}} {\text{= a(1/C}}_{\text{i}} \text{) + b}$

(i = 1, 2 ...m, m ≧ 2, where i = 1 is a first block)
Then, the CPU 34 operates index R_{MAX} in accordance with the following operation expression, to store the same in the storage area 8ℓ1 of the RAM 35:

${\text{R}}_{\text{MAX}} \text{= 1/b}$

In other words, said means for obtaining an index (R_{MAX}) expressing the total amount of hepatic cell function includes means for injecting said specific dye, dividing a prescribed time interval from uniform distribution of said specific dye in said blood into a plurality of blocks to obtain coefficients Aᵢ and Bᵢ on the basis of simulation functions 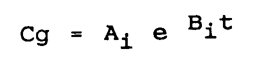
(i = 1, 2, ..., m, m = 2, where i = 1 is a first block) in respective said blocks and to obtain values of Cg in initial times of respective said blocks as Cᵢ assuming that ${\text{k}}_{\text{i}} {\text{= - B}}_{\text{i}}$ and performing regression line analysis on the basis of obtained said coefficients kᵢ and Cᵢ by an operation expression of ${\text{(1/k}}_{\text{i}} {\text{) = a(1/C}}_{\text{i}} \text{) + b}$ to obtain coefficients a and b, thereby to obtain said index R_{MAX} on the basis of an operation expression of ${\text{R}}_{\text{MAX}} \text{= 1/b}$ .

Although three time blocks are provided in the above embodiment, such time blocks may be in any number so far as the same is at least two, and the accuracy is improved as the number of time blocks is increased.

Although 1/Cg₁, 1/Cg₂ and 1/Cg₃ are plotted in the axis of abscissa, this is a simplified type and index R_{MAX} can be more correctly measured by evaluating the coefficient A₁ on the basis of the following operation expression to assume the coefficient A₁ as a coefficient C₀₁, and similarly evaluating coefficients C₀₂ and C₀₃ to create the data as shown in Fig. 22. Assuming that T₁ = 5 min. and the dose of ICG is D₁ mg/kg, CO₁ may correspond to D₁, D₂ may be equal to D₁ x CO₂/CO₁ and D₃ may be equal to D₁ x CO₁/CO₃. D₁ may be previously set at 2 mg/kg, for example, as a value specific to the apparatus, or may be inputted by connecting input means to the CPU 34.

In other words, said means for obtaining an index (r_{MAX}) expressing the total amount of hepatic all function includes means for injecting said specific dye, dividing a prescribed time interval from uniform distribution of said specific dye in said blood into a plurality of blocks to obtain coefficients Aᵢ and Bᵢ on the basis of simulation functions of 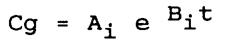
(i = 1, 2, ..., m, m = 2, where i = 1 is a first block) in respective said blocks and to obtain, on the basis of obtained said coefficients Aᵢ and load quantity D₁ of said specific dye, Dᵢ by an operation expression of ${\text{D}}_{\text{i}} {\text{= D₁ x A}}_{\text{i}} \text{/A₁}$ 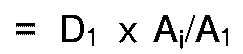 assuming that ${\text{k}}_{\text{i}} {\text{= - B}}_{\text{i}}$ and performing regression line analysis from an operation expression of ${\text{(1/k}}_{\text{i}} {\text{) = c (1/D}}_{\text{i}} \text{) + d}$ on the basis of obtained Kᵢ and Dᵢ to obtain coefficients c and d, thereby to obtain said index r_{MAX} from r_{MAX} = 1/d.

The CPU 34, having operated index r_{MAX} stores the same in the storage area 8ℓ2 of the RAM 35.

According to the present invention as hereinabove described, vital tissue is exposed to first light of a wavelength absorbed by specific dye dosed into the blood of the vital tissue to be taken in and removed by the liver and second light of a wavelength not absorbed by the dye and first and second photoelectric conversion signals corresponding to the first light and the second light obtained from the vital tissue are sampled so that the coefficient of a regression line expression between the first and second photoelectric conversion signals is determined on the basis of variable components in the blood included in the sampled first and second photoelectric conversion signals, thereby to operate a value correlated with specific dye concentration in the blood on the basis of a sampling signal during a prescribed period after a lapse of a predetermined time from injection of the specific dye and the determined coefficient of the regression line expression. Thus, the value correlated with the specific dye concentration is operated to remove artifacts caused by blood flow disturbance and vibration of an organism in attachment of a sensor to the organism by performing biocalibration, thereby to more correctly test/diagnose the liver function.

### INDUSTRIAL APPLICABILITY

The liver function testing apparatus according to the present invention is employed as an apparatus for testing/diagnosing liver function by performing biocalibration before injecting specific dye selectively taken in and removed by the liver into the blood for removing artifacts and thereafter injecting the specific dye into the blood to more correctly measure the blood plasma disappearance rate and the retention rate.

## Claims

1. A liver function testing apparatus for testing liver function, comprising:
- light source means (11, 22) for exposing vital tissue to first light of a wavelength absorbed by specific dye dosed into blood of said vital tissue to be taken in and removed by the liver and second light of a wavelength not absorbed by said specific dye;
- photoelectric conversion means (13) for outputting first and second photoelectric conversion signals (L₁,L₂) corresponding to said first light and said second light applied to said vital tissue by said light source means and obtained from said vital tissue; and
characterized by sampling means (28) for sampling said first and second photoelectric conversion signals (L₁,L₂) a plurality of times;
- decision means (34) for deciding a correlation coefficient (r₁) of a regression line expression between said first and second photoelectric conversion signals on the basis of variable components in said blood included in said first and second photoelectric conversion signals sampled by said sampling means, including means for obtaining constants A and B by performing the regression line analysis in accordance with the following operation expression:
$\text{logCL₁ = A · logCL₂ + B}$
assuming that CL₁ and CL₂ represent sampled values of said first and second photoelectric conversion signals sampled by said sampling means a plurality of times in a calibration mode, and obtaining a value CL₁₀ being the maximum value of CL₁
- arithmetic means for operating a value Cg(I) correlated with specific dye concentration, in said blood on the basis of said constants A and B, the maximum value CL₁₀ and the conversion signals L₁(I), and L₂(I) in accordance with the following operation expression: 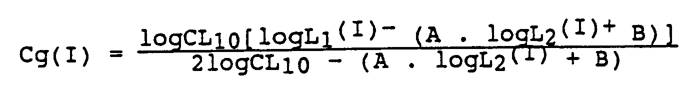 for discrete sample paints (I) during a prescribed period in a measurement mode from injection of said specific dye if said correlation coefficient of said regression line expression is greater than a predetermined value.

2. A liver function testing apparatus in accordance with claim 1, further including coefficient operating means for obtaining a correlation coefficient (r₂) of a simulation function given by: 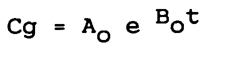 assuming that t represents said prescribed period after injection of said specific dye by using the method of least squares on the basis of said values C_{g}(I) correlated with said specific dye concentration operated by said arithmetic means.

3. A liver function testing apparatus in accordance with claim 2, further including means for obtaining a blood plasma disappearance rate (k) of said specific dye on the basis of said simulation function from the following operation expression:
${\text{k = -B}}_{\text{o}} \text{.}$

4. A liver function testing apparatus in accordance with claim 3, further including means (37) for outputting said blood plasma disappearance rate (k) obtained by said means for obtaining said blood plasma disappearance rate.

5. A liver function testing apparatus in accordance with claim 2, further including means for obtaining a retention rate (R%) of said specific dye in said prescribed time on the basis of said simulation function from the following operation expression:

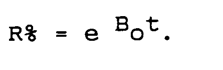

6. A liver function testing apparatus in accordance with claim 5, further including means (37) for outputting said retention rate (R%) obtained by said means for obtaining said retention rate.

7. A liver function testing apparatus in accordance with claim 2, further including means for obtaining an index (R_{MAX}) expressing the total amount of hepatic cell function; wherein said means for obtaining said index R_{MAX} includes means for injecting said specific dye, dividing a prescribed time interval from uniform distribution of said specific dye in said blood into a plurality of blocks to obtain coefficients Aᵢ and Bᵢ on the basis of simulation functions 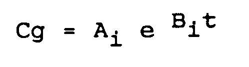 (i = 1, 2, ..., m, m = 2, where i = 1 is a first block) in respective said blocks and to obtain values of Cg in initial times of respective said blocks as Cᵢ assuming that ${\text{k}}_{\text{i}} {\text{= - B}}_{\text{i}}$ and performing regression line analysis on the basis of obtained said coefficients kᵢ and Cᵢ by an operation expression of ${\text{(1/k}}_{\text{i}} {\text{) = a(1/C}}_{\text{i}} \text{) + b}$ 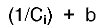 to obtain coefficients a and b, thereby to obtain said index R_{MAX} on the basis of an operation expression of ${\text{R}}_{\text{MAX}} \text{= 1/b}$ .

8. A liver function testing apparatus in accordance with claim 2, further including means for obtaining an index (r_{MAX}) expressing the total amount of hepatic all function, wherein said means for obtaining said index r_{MAX} includes means for injecting said specific dye, dividing a prescribed time interval from uniform distribution of said specific dye in said blood into a plurality of blocks to obtain coefficients Aᵢ and Bᵢ on the basis of simulation functions of 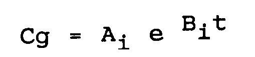 (i = 1, 2, ..., m, m = 2, where i = 1 is a first block) in respective said blocks and to obtain, on the basis of obtained said coefficients Aᵢ and load quantity D₁ of said specific dye, Dᵢ by an operation expression of ${\text{D}}_{\text{i}} {\text{= D₁ x A}}_{\text{i}} \text{/A₁}$ assuming that ${\text{k}}_{\text{i}} {\text{= - B}}_{\text{i}}$ and performing regression line analysis from an operation expression of ${\text{(1/k}}_{\text{i}} {\text{) = c (1/D}}_{\text{i}} \text{) + d}$ on the basis of obtained Kᵢ and Dᵢ to obtain coefficients c and d, thereby to obtain said index r_{MAX} from ${\text{r}}_{\text{MAX}} \text{= 1/d}$ .

9. A liver function testing apparatus in accordance with claim 1, further including informing means (40) for giving an alarm when said correlation coefficient (r₁) operated by said means for operating said correlation coefficient is greater than a predetermined value.

10. A liver function testing apparatus in accordance with claim 2, further including informing means (40) for giving an alarm when said correlation coefficient (r₂) of said simulation function is greater than a predetermined value.

11. A liver function testing apparatus in accordance with claim 1, further including mode selection means (41, 43) for selecting a biocalibration mode for performing operation for deciding said coefficient of said regression line expression by said decision means and a measurement mode for performing operation for operating said value correlated with said specific dye concentration by said arithmetic means.

12. A liver function testing apparatus in accordance with claim 11, further including means (42) for activating said decision means in response to selection of said biocalibration mode by said mode selection means.

13. A liver function testing apparatus in accordance with claim 11, further including means (42) for activating said arithmetic means in response to selection of said measurement mode by said mode selection means.

14. A liver function testing apparatus in accordance with claim 1, further including set means for setting intensity levels of said first light and said second light emitted from said light source means so that levels of said first and second photoelectric conversion signals are within a predetermined range.

15. A liver function testing apparatus in accordance with claim 1, further including indication means (33) for indicating timing for injecting said specific dye into said blood.

## Patentansprüche

1. Leberfunktions-Testeinrichtung zum Testen der Leberfunktion, die umfaßt:
- Lichtquellenmittel (11, 22) zur Belichtung von Lebendgewebe mit einer ersten Lichtmenge einer Wellenlänge, die von einem Kennzeichnungsfarbstoff absorbiert wird, der in das Blut des Lebendgewebes eingeführt wird, um von der Leber aufgenommen und entfernt zu werden, und mit einer zweiten Lichtmenge einer Wellenlänge, die nicht von dem Kennzeichnungsfarbstoff absorbiert wird;
- photoelektrische Umwandlungsmittel (13) zur Ausgabe von ersten und zweiten photoelektrischen Umwand-lungssignalen (L₁, L₂), die der ersten Lichtmenge und der zweiten Lichtmenge entsprechen, die auf das Lebendgewebe mit dem Lichtquellenmittel gerichtet wurden und von dem Lebendgewebe erhalten wurden;
**gekennzeichnet durch** Abtastmittel (28) zum vielfachen Abtasten der ersten und zweiten photoelektrischen Umwandlungssignale (L₁, L₂);
- Bestimmungsmittel (34) zur Bestimmung eines Korrelationskoeffizienten (r₁) eines Regressionskurvenausdruckes zwischen den ersten und zweiten photoelektrischen Umwandlungssignalen auf Grundlage von variablen Komponenten im Blut, die in den ersten und zweiten photoelektrischen Umwandlungssignalen enthalten sind, die mit dem Abtastmittel abgetastet wurden, welches Mittel zur Ermittlung von konstanten A und B unter Durchführung einer Regressionskurvenanalyse gemäß dem folgenden Operationsausdruck umfaßt:
$\text{logCL₁ = A · logCl₂ + B}$
wobei vorausgesetzt wird, daß CL₁ und CL₂ abgetastete Werte der ersten und zweiten photoelektrischen Umwandlungssignale darstellen, die mit dem Abtastmittel in einem Kalibrierungsmodus vielfach abgetastet wurden, wobei ein Wert CL₁₀ ermittelt wird, der den Maximalwert von CLᵢ darstellt,
- Berechnungsmittel zur Bestimmung eines Wertes Cg (r), der mit der Konzentration des Kennzeichnungsfarbstoffes im Blut auf Grundlage der Konstanten A und B, des Maximalwertes CL₁₀ und der Umwandlungssignale L₁(I) und L₂(I) entsprechend dem folgenden Operationsausdruck: 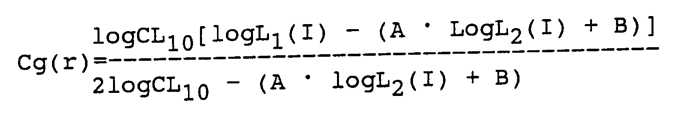 für diskrete Abtastpunkte (I) während eines vorbestimmten Zeitbereiches in einem Meßmodus nach Injektion des Kennzeichnungsfarbstoffes korreliert ist, wenn der Korrelationskoeffizient des Regressionskurvenausdruckes größer als ein vorbestimmter Wert ist.

2. Leberfunktions-Testeinrichtung nach Anspruch 1, die weiterhin Koeffizienten-Berechnungsmittel zum Ermitteln eines Korrelationskoeffizienten (r₂) einer durch 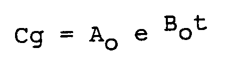 gegebenen Simulationsfunktion enthält, wobei angenommen wird, daß t den vorbestimmten Zeitbereich nach Injektion des Kennzeichnungsfarbstoff darstellt und wobei das Verfahren der kleinsten Fehlerquadrate auf Grundlage der Werte Cg(I) verwendet wird, die mit der Konzentration des Kennzeichnungsfarbstoff korreliert sind, welche durch das Arithmetikmittel berechnet werden.

3. Leberfunktions-Testeinrichtung nach Anspruch 2, die weiterhin Mittel zur Ermittlung einer Blutplasma-Abbaurate (k) des Kennzeichnungsfarbstoff auf Grundlage der simulationsfunktion gemäß dem folgenden Operationsausdruck enthält: ${\text{k = -B}}_{\text{o}}$ .

4. Leberfunktions-Testeinrichtung nach Anspruch 3, die weiterhin Mittel (37) zur Ausgabe der Blutplasma-Abbaurate (k) enthält, die mit den Mitteln zur Ermittlung der Blutplasma-Abbaurate erhalten wurde.

5. Leberfunktions-Testeinrichtung nach Anspruch 2, die weiterhin Mittel zur Ermittlung einer Zurückhalterate (R %) des KennzeichnungsfarbstoffeS in der vorbestimmten Zeit auf Grundlage der Simulationsfunktion entsprechend dem folgenden Operationsausdruck enthält:

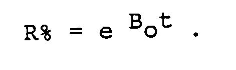

6. Leberfunktions-Testeinrichtung nach Anspruch 5, die weiterhin Mittel (37) zur Ausgabe der Zurückhalterate (R%) enthält, die mit dem Mittel zur Ermittlung der Zurückhalterate erhalten wurde.

7. Leberfunktions-Testeinrichtung nach Anspruch 2, die weiterhin Mittel zur Ermittlung einer Kennzahl (r_{MAX}) enthält, die den Gesamtbetrag der Leberzellfunktion ausdrückt, wobei das Mittel zur Ermittlung der Kennzahl R_{MAX} Mittel zur Injektion des KennzeichnungsfarbstoffeS enthält, das ein vorbestimmtes Zeitintervall gleichförmiger Verteilung des KennzeichnungsfarbstoffeS in dem Blut in eine Vielzahl von Blöcken unterteilt, um auf Grundlage von Simulationsfunktionen 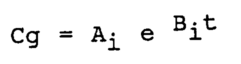 (i = 1), 2, ..., m, wobei m = 2, wenn i = 1 einen ersten Block darstellt), Koeffizienten Aᵢ und Bᵢ in den entsprechenden Blöcken zu ermitteln und um als Cᵢ Werte von Cg zu Startzeiten der entsprechenden Blöcke zu ermitteln, wobei angenommen wird, daß ${\text{k}}_{\text{i}} {\text{= -B}}_{\text{i}}$ beträgt, und wobei eine Regressionskurvenanalyse auf Grundlage der erhaltenen Koeffizienten kᵢ und Cᵢ mit einem Operationsausdruck gemäß ( ${\text{1/k}}_{\text{i}} {\text{) = a(1/C}}_{\text{i}} \text{) + b}$ durchgeführt wird, um Koeffizienten a und b zu erhalten, um dadurch die Kennzahl r_{MAX} auf Grundlage eines Operationsausdruckes gemäß ${\text{R}}_{\text{MAX}} \text{= 1/b}$ zu ermitteln.

8. Leberfunktions-Testeinrichtung nach Anspruch 2, die weiterhin Mittel zur Ermittlung einer Kennzahl (r_{MAX}) enthält, die den Gesamtbetrag einer Leberzellfunktion ausdrückt, wobei das Mittel zur Ermittlung der Kennzahl r_{MAX} Mittel zur Injektion des Kennzeichnungsfarbstoffes enthält und ein vorbestimmtes Zeitintervall der gleichförmigen Verteilung des Kennzeichnungsfarbstoffes in dem Blut in eine Vielzahl von Blöcken unterteilt, um Koeffizienten Aᵢ und Bᵢ auf Grundlage der Simulationsfunktionen gemäß 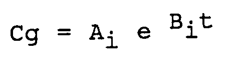 (i = 1), 2, ..., m, wobei m = 2, wenn i = 1 einen ersten Block darstellt), in den entsprechenden Blöcken zu ermitteln und um auf Grundlage des Koeffizienten Aᵢ und der Lademenge D₁ des Kennzeichnungsfarbstoffes Dᵢ mit einem Operationsausdruck gemäß ${\text{D}}_{\text{i}} {\text{= D₁ * A}}_{\text{i}} \text{/A₁}$ zu ermitteln, wobei angenommen wird, daß ${\text{k}}_{\text{i}} {\text{= -B}}_{\text{i}}$ beträgt, und wobei eine Regressionskurvenanalyse aus einem Operationsausdruck gemäß ${\text{(1/k}}_{\text{i}} {\text{) = c (1/D}}_{\text{i}} \text{) + d}$ auf Grundlage der ermittelten Kᵢ und Dᵢ durchgeführt wird, um Koeffizienten c und d zu erhalten, um dabei die Kennzahl r_{MAX} gemäß ${\text{r}}_{\text{MAX}} \text{= 1/d}$ zu ermitteln.

9. Leberfunktions-Testeinrichtung nach Anspruch 1, die weiterhin Informationsmittel (40) zur Abgabe einer Warnung enthält, wenn der Korrelationskoeffizient (r₁), der von dem Mittel zur Berechnung des Korrelationskoeffizienten berechnet wird, größer als ein vorbestimmter Wert ist.

10. Leberfunktions-Testeinrichtung nach Anspruch 2, die weiterhin Informationsmittel (40) zur Abgabe einer Warnung enthält, wenn der Korrelationskoeffizient (r₂) der Simulationsfunktion größer als ein vorbestimmter Wert ist.

11. Leberfunktions-Testeinrichtung nach Anspruch 1, die weiterhin Betriebsauswahlmittel (41, 43) zur Auswahl eines Biokalibrierungsmodus zur Durchführung des Betriebsablaufes zur Bestimmung des Koeffizienten des Regressionskurvenausdruckes mit dem Bestimmungsmittel und eines Meßmodus zur Durchführung des Betriebsablaufes des mit der Konzentration des Kennzeichnungsfarbstoffes korrelierten Wertes mit dem Arithmetikmittel enthält.

12. Leberfunktions-Testeinrichtung nach Anspruch 11, die weiterhin Mittel (42) zur Aktivierung des Bestimmungsmittels in Reaktion auf die Anwahl des Biokalibrierungsmodus mit dem Betriebswahlmittel enthält.

13. Leberfunktions-Testeinrichtung nach Anspruch 11, die weiterhin Mittel (42) zur Aktivierung des Arithmetikmittels in Reaktion auf die Anwahl des Meßmodus mit dem Betriebswahlmittel enthält.

14. Leberfunktions-Testeinrichtung nach Anspruch 1, die weiterhin Einstellmittel zur Einstellung von Intensitätspegeln der ersten Lichtmenge und der zweiten Lichtmenge enthält, die von den Lichtquellenmitteln emittiert werden, so daß die Pegel der ersten und zweiten photoelektrischen Umwandlungssignale innerhalb eines vorbestimmten Bereiches liegen.

15. Leberfunktions-Testeinrichtung nach Anspruch 1, die weiterhin Kennzeichnungsmittel (33) zur Kennzeichnung des Zeitablaufes zur Injektion des Kennzeichnungsfarbstoffes in das Blut enthält.

## Revendications

1. Appareil de contrôle des fonctions hépatiques pour contrôler certaines fonctions hépatiques comprenant:
- un moyen de source de lumière (11, 22) pour exposer des tissus vivants à une première lumière dont la longueur d'onde est absorbée par un colorant spécifique dosé dans le sang dudit tissus vital, pour être capté et éliminé par le foie, et à une seconde lumière dont la longueur d'onde n'est pas absorbée par ledit colorant spécifique;
- un moyen de conversion photoélectrique (13) pour produire un premier et un deuxième signaux de conversion photoélectrique (L₁, L₂) correspondant à ladite première lumière et à ladite seconde lumière appliquées audit tissu vital par ledit moyen de source de lumière et provenant dudit tissu vital; et
caractérisé par un moyen d'échantillonnage (28) pour échantillonner plusieurs fois lesdits premier et deuxième signaux de conversion photoélectrique (L₁-L₂);
- un moyen de décision (34) pour décider du coefficient de corrélation (r₁) d'une expression de régression linéaire entre lesdits premier et deuxième signaux de conversion photoélectrique en fonction de constituants variables dans ledit sang inclus dans lesdits premier et deuxième signaux de conversion photoélectrique échantillonnés par ledit moyen d'échantillonnage, comportant un moyen pour obtenir des constantes A et B par une analyse de régression linéaire effectuée conformément à l'expression mathématique suivante: 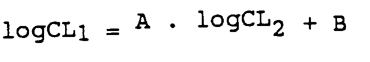 en supposant que CL₁ et CL₂ représentent des valeurs échantillonnées desdits premier et deuxième signaux de conversion photoélectrique échantillonnés plusieurs fois par ledit moyen d'échantillonnage en mode d'étalonnage, et en déterminant une valeur CL10 comme étant la valeur maximale de CL₁,
- un moyen arithmétique pour calculer une valeur Cg(I) corrélée avec une concentration spécifique en colorant dans ledit sang, en fonction desdites constantes A et B, de la valeur maximale CL₁₀ et des signaux de conversion L₁(I) et L₂(I), conformément à l'expression mathématique suivante: 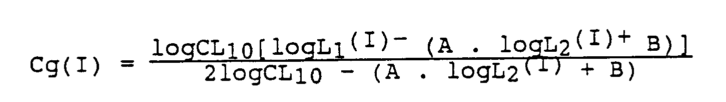 pour des points d'échantillonnage discrets pendant une période prédéterminée en mode de mesure, à partir de l'injection dudit colorant spécifique, si ledit coefficient de corrélation de ladite expression de régression linéaire, est supérieur à une valeur prédéterminée.

2. Appareil de contrôle des fonctions hépatiques selon la revendication 1, comportant en outre un moyen de calcul de coefficient, pour obtenir le coefficient de corrélation (r₂) d'une fonction de simulation donnée par: 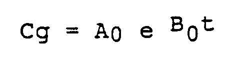 en supposant que t représente ledit temps prédéterminé à partir de l'injection dudit colorant spécifique, en appliquant la méthode des moindres carrés auxdites valeurs Cg(I) corrélées à ladite concentration spécifique en colorant calculée par ledit moyen arithmétique.

3. Appareil de contrôle des fonctions hépatiques selon la revendication 2, comportant en outre un moyen pour obtenir le coefficient d'épuration plasmatique sanguin (k) dudit colorant spécifique sur la base de ladite fonction de simulation, à partir de l'expression mathématique suivante:
$\text{k = - B₀}$ .

4. Appareil de contrôle des fonctions hépatiques selon la revendication 3, comportant en outre un moyen (37) pour fournir ledit coefficient d'épuration plasmatique sanguin (k) obtenu par ledit moyen, afin d'obtenir ledit coefficient d'épuration plasmatique sanguin.

5. Appareil de contrôle des fonctions hépatiques selon la revendication 2, comportant en outre un moyen pour obtenir le taux de rétention (R%) dudit colorant spécifique au cours dudit temps prédéterminé, sur la base de ladite fonction de simulation, à partir de l'expression mathématique suivante:

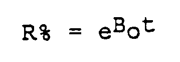

6. Appareil de contrôle des fonctions hépatiques selon la revendication 1, comportant en outre un moyen (37) pour produire ledit taux de rétention (R%) obtenu par ledit moyen d'obtention dudit taux de rétention.

7. Appareil de contrôle des fonctions hépatiques selon la revendication 2, comportant en outre un moyen pour obtenir un indice (R_{MAX}) exprimant la quantité totale des fonctions cellulaires hépatiques; dans lequel ledit moyen d'obtention dudit indice (R_{MAX}) comporte un moyen pour injecter ledit colorant spécifique, diviser un intervalle de temps prédéterminé partant de l'instant où la répartition dudit colorant spécifique dans ledit sang est uniforme, en une pluralité de blocs afin d'obtenir des coefficients Aᵢ et Bᵢ sur la base de fonctions de simulation de la forme 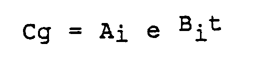 (i = 1, 2, ..., m, m = 2, où i = 1 s'il s'agit d'un premier bloc) dans lesdits blocs respectifs afin d'obtenir des valeurs de Cg aux instants initiaux des blocs respectifs en tant que valeurs Cᵢ, en supposant que ${\text{k}}_{\text{i}} {\text{= -B}}_{\text{i}}$ et en effectuant une analyse par régression linéaire sur la base desdits coefficients kᵢ et Cᵢ obtenus par une expression mathématique de la forme ${\text{(1/k}}_{\text{i}} {\text{) = a(1/C}}_{\text{i}} \text{) + b}$ , afin d'obtenir des coefficients a et b et ainsi, d'obtenir ledit indice R_{MAX} sur la base d'une expression mathématique de la forme ${\text{R}}_{\text{MAX}} \text{= 1/b}$ .

8. Appareil de contrôle des fonctions hépatiques selon la revendication 2, comportant en outre un moyen pour obtenir un indice (r_{MAX}) exprimant la totalité des fonctions hépatiques, dans lequel ledit moyen d'obtention dudit indice r_{MAX} comporte un moyen pour injecter ledit colorant spécifique, pour diviser un intervalle de temps prédéterminé partant de l'instant où la répartition dudit colorant spécifique dans ledit sang est uniforme, en une pluralité de blocs permettant d'obtenir des coefficients Aᵢ et Bᵢ sur la base de fonctions de simulation de la forme 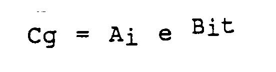 (i = 1, 2, ..., m, m = 2, où i = 1 s'il s'agit d'un premier bloc), dans lesdits blocs respectifs, afin d'obtenir, sur la base desdits coefficients obtenus Aᵢ et de la quantité introduite D₁ dudit colorant spécifique, les valeurs de Dᵢ à partir d'une expression mathématique de la forme ${\text{D}}_{\text{i}} {\text{= D₁ x A}}_{\text{i}} \text{/ A₁}$ en supposant que ${\text{k}}_{\text{i}} {\text{= - B}}_{\text{i}}$ et en effectuant une analyse par régression linéaire à partir d'une expression mathématique de la forme ${\text{(1/k}}_{\text{i}} {\text{) = c (1/D}}_{\text{i}} \text{) + d}$ , sur la base des coefficients Kᵢ et Dᵢ obtenus, afin d'obtenir des coefficients c et d, et ainsi, d'obtenir l'indice r_{MAX} à partir de la relation ${\text{r}}_{\text{MAX}} \text{= 1/d}$ .

9. Appareil de contrôle des fonctions hépatiques selon la revendication 1, comportant en outre un moyen d'information (40) pour produire une alarme lorsque ledit coefficient de corrélation (r₁) calculé par ledit moyen de calcul dudit coefficient de corrélation, est supérieur à une valeur prédéterminée.

10. Appareil de contrôle des fonctions hépatiques selon la revendication 2, comportant en outre un moyen d'information (40) pour produire une alarme lorsque ledit coefficient de corrélation (r₂) de ladite fonction de simulation est supérieur à une valeur prédéterminée.

11. Appareil de contrôle des fonctions hépatiques selon la revendication 1, comportant en outre un moyen de sélection de mode (41, 43) pour sélectionner un mode de bio-étalonnage pour que ledit moyen de décision effectue une opération de décision dudit coefficient de ladite droite de régression, et un mode de mesure pour que ledit moyen arithmétique effectue une opération de calcul de ladite valeur corrélée à ladite concentration spécifique en colorant.

12. Appareil de contrôle des fonctions hépatiques selon la revendication 11, comportant en outre un moyen (42) pour activer ledit moyen de décision en réponse à la sélection dudit mode de bio-étalonnage par ledit moyen de sélection de mode.

13. Appareil de contrôle des fonctions hépatiques selon la revendication 11, comportant en outre un moyen (42) pour activer ledit moyen arithmétique en réponse à la sélection dudit mode de mesure par ledit moyen de sélection de mode.

14. Appareil de contrôle des fonctions hépatiques selon la revendication 1, comportant en outre un moyen de réglage pour régler des niveaux d'intensité de ladite première lumière et de ladite seconde lumière émises par ledit moyen de source de lumière de façon à ce que les niveaux desdits premier et deuxième signaux de conversion photoélectrique se situent dans une gamme prédéterminée.

15. Appareil de contrôle des fonctions hépatiques selon la revendication 1, comportant en outre un moyen d'indication (33) pour indiquer l'instant où ledit colorant spécifique doit être injecté dans ledit sang.
